(19) Europäisches Patentamt
European Patent Office
Office européen des brevets

(11) **EP 2 038 439 B3**

(12) **NEW EUROPEAN PATENT SPECIFICATION**
After limitation procedure (B3-1)

(45) Mention of the grant of the patent:
**07.09.2011 Bulletin 2011/36**

(45) Date of publication and mention
of the limitation decision:
**B3-1 11.11.2020 Bulletin 2020/46**

(21) Application number: **07799238.6**

(22) Date of filing: **02.07.2007**

(51) Int Cl.:
*C22B 9/00* (2006.01)      *C22B 26/22* (2006.01)
*C07C 19/08* (2006.01)      *C07C 21/18* (2006.01)

(86) International application number:
**PCT/US2007/072643**

(87) International publication number:
**WO 2008/005920 (10.01.2008 Gazette 2008/02)**

(54) **COVER GAS COMPOSITION FOR MOLTEN NON-FERROUS METALS SUCH AS MAGNESIUM**

SCHUTZGASZUSAMMENSETZUNG FÜR GESCHMOLZENE NICHTEISENMETALLE WIE MAGNESIUM

GAZ DE COUVERTURE POUR POUR MÉTAUX NON FERREUX EN FUSION TEL QUE LE MAGNÉSIUM

(84) Designated Contracting States:
**DE ES FR GB IT**

(30) Priority: **03.07.2006 US 818416 P**
**26.03.2007 US 691276**

(43) Date of publication of application:
**25.03.2009 Bulletin 2009/13**

(73) Proprietor: **Honeywell International Inc.**
**Morristown, NJ 07960 (US)**

(72) Inventors:
• **LULY, Matthew H.**
**Hamburg, New York 14075 (US)**

• **SINGH, Rajiv R.**
**Getzville, New York 14068 (US)**

(74) Representative: **Crooks, Elizabeth Caroline**
**Kilburn & Strode LLP**
**Lacon London**
**84 Theobalds Road**
**London WC1X 8NL (GB)**

(56) References cited:
**EP-B1- 0 964 845        WO-A-01/83836**
**WO-A-2004/037752     US-A- 5 710 352**
**US-B1- 6 929 674**

**Description**

**BACKGROUND OF THE INVENTION**

**(1) Field of Invention**

The present invention relates to cover gas compositions for molten nonferrous metal, such as magnesium, and methods of using the same to prevent the oxidation when the metal is exposed to air.

**(2) Description of Related Art**

Certain non-ferrous metals, such as magnesium, aluminuin, and lithium, are highly reactive and oxidatively unstable. For example, molten magnesium is readily and violently oxidized in ambient air or dry air, burning with a flame temperature of approximately 2820°C. Three approaches have been suggested to inhibit these severe oxidation processes: (1) sprinkling salt cover fluxes over the molten metal; (2) excluding oxygen from contacting the molten metal by blanketing the molten metal with an inert gas such as helium, nitrogen or argon; or (3) blanketing the molten metal with a protective cover gas composition. Projective cover gas compositions typically comprise air and/or carbon dioxide and a small amount of an inhibiting agent which reacts or interacts with the molten metal to form a film or layer on the molten metal surface which protects it from oxidation.

US 1,972,317 (Reimers) relates to methods for inhibiting the oxidation of readily oxidizable metals, including magnesium and its alloys. Reimers notes that at the time of its filing in 1932, numerous solutions had been proposed to the oxidation problem including displacing the atmosphere in contact with the metal with a gas such as nitrogen, carbon dioxide, or sulfur dioxide. Reimers teaches inhibition of oxidation by maintaining in the atmosphere in contact with molten metal an inhibiting gas containing fluorine, either in elemental or combined form. Reference is made to many fluorine containing compounds with the solids ammonium borofluoride, ammonium silicofluoride, ammonium bifluoride and ammonium fluophosphate or the gases evolved therefrom upon heating being said to be preferred. Not with standing the disclosure in Reimers, it was not until about the mid-1970's that a fluorine containing compound found commercial acceptance as an inhibiting agent in a cover gas.

Prior to about the mid-1970's, sulfur dioxide ($SO_2$) was widely used as an inhibiting agent in a magnesium cover gas composition. However, $SO_2$ was subsequently replaced by sulfurhexafluoride ($SF_6$) which is currently the industry standard. Typically, $SF_6$ based cover gas compositions contain 0.2-1% by volume $SF_6$ and a carrier gas such as air, carbon dioxide, argon, or nitrogen. $SF_6$ has the advantages that it is a colorless, odorless, non-toxic gas which can be used for protecting molten magnesium/magnesium alloy and in the production of bright and shiny ingots with relatively low dross formation. However, $SF_6$ suffers from several disadvantages, including: its sulfur-based decomposition products at high temperature are very toxic; it is expensive and has limited sources of supply; and it is a known greenhouse gas having, at a time horizon of 100 years, a Global Warming Potential (GWP) of 23,900 relative to 1 for carbon dioxide.

It is also noted that once magnesium has ignited, the resulting fire cannot be extinguished even with high concentrations of $SF_6$. The potential by product SO, is even worse in this respect as it can accelerate a magnesium fire.

Another cover gas useful for extinguishing a magnesium fire is boron trifluoride ($BF_3$). However, this material tends to be very expensive and is also very toxic.

The problem of GWP of cover gases has been addressed in WO 00/64614 wherein certain relatively low GWP hydrofluorocarbons and hydrofluoroethers such as difluoromethane (HFC-32), pentafluoroethane (HFC-125), 1,1,1,2-tetrafluoroethane (HFC-134a), difluoroethane (HFC-152a), methoxy-nonafluorobutane (HFE-7100), ethoxy-nonafluorobutane (HFE-7200), and others were disclosed as being useful as blanket gases for protecting molten magnesium and magnesium alloys from Oxidation. US 6,521,018 (Hobbs) also discloses certain low GWP compounds that may be useful as blanket gases for nonferrous metals and alloys including, carbonyl fluoride ($COF_2$), trifluoroacetyl fluoride ($CF_3COF$), 1,1,1,3,3,3-hexafluoropropan-2-one (($CF_3)_2CO$), nitrogen trifluoride ($NF_3$), sulfuryl fluoride ($SO_2F_2$), nitrosyl fluoride (NOF), fluorine gas ($F_2$), and others. Still other compounds useful for magnesium blanket gases are disclosed in US 6,537,346, US 6,685,764, and US 6,780,220 (all by Milbrath), including perfluoroketones such as $C_2F_5C(O)CF(CF_3)_2$.

Although previously suggested compounds may have certain limited utility as cover gases, alternative cover gas compositions thai have superior characteristics, such as a low GWP, low boiling point, uniform dispersement, and low or no toxicity, are desirable. Applicants have discovered that certain fluoroolefins, such as for example, $CF_3CH=CHF$ (*trans*-HFO- 1234ze), are useful as cover gases for nonferrous reactive metals.

Applicants discovery is contrary, in at least some respects, to prior teachings. For example, it has heretofore been believed that fluoroolefins are undesirable as cover gases due to environmental and/or toxicity concerns, (See e.g. D. Milbrath, "Development of 3M Novec 612 Magnesium Protection Fluid as a Substitute for SF6 over Molten Magnesium", International Conference on SF6 and the Environment, Nov. 21-22, 2002, www.epa.gov/highgwp/elec tricpowerw-sf6/pdf/milbrath.pdf.)

US Patent No. 6,929,674 discloses a cover gas composition for protecting molten magnesium/magnesium alloy. The composition comprises a fluorine containing inhibiting agent selected from the group consisting of difluoromethane, pentafluoroethane, 1,1,1,2-tetrafluoroethane, difluoromethane, heptafluoropropane, dihydrodecafluoropentane, hydrofluoroethers and mixtures thereof, and a carrier gas, wherein each component of the composition has a Global Warming Potential (GWP)(referenced to the absolute GWP for carbon dioxide at a time horizon of 100 years) of less than 5000.

European Patent No. 0 964 845 discloses a process for producing the pentafluoropropenes of the formula $CF_3CX=CF_2$, where X is H or Cl, the process comprising hydrohalogenating $CF_3CCl_2CF_3$ with hydrogen at an elevated temperature in the vapor phase over a catalyst comprising at least one component selected from the group consisting of elemental metals, metal oxides, metal halides and metal oxyhalides; wherein the metal of said hydrohalogenation catalyst component is selected from copper, nickel, chromium and mixtures thereof and the halogen of said halides and said oxyhalides is selected from fluorine, chlorine and mixtures thereof.

US Patent No. 5,710,352 discloses a method for the preparation of 1,1,1,3,3-pentafluoropropane (HFC-245fa) and 1-chloro-3,3,3-trifluoropropene (HCFC-1233). The method for the preparation of 1,1,1,3,3-pentafluoropropane (HFC-245fa) comprises fluorinating 1,1,1,3,3-pentachloropropane (HCC-240fa) with hydrogen fluoride in a vapor phase in the presence of a fluorination catalyst, thereby forming a reaction product comprising HCl, 1,1,1,3,3-pentafluoropropane, 1-chloro-3,3,3-trifluoropropene (HCFC-1233) and 1,3,3,3-tetrafluoropropene (HFC-1234). The HCFC-1233 and any co-produced 1,3,3,3-tetrafluoropropene (HFC-1234) are recycled for further fluorination by HF for a greater than 99% HCC-240fa conversion.

WO 2004/037752 discloses azeotrope-like compositions comprising pentafluoropropenes (HFO-1225) and a fluid selected from the group consisting of 3,3,3-trifluoropropene (HFO-1234zf), 1,1-difluoroethane (HFC-152A), trans-1,3,3,3-tetrafluoropropene (HFO-1234ze), and combinations of two or more thereof. Also provided are uses thereof including as refrigerants, blowing agents, sprayable compositions, flame suppressant, and the like.

## SUMMARY OF THE INVENTION

One aspect of the present invention provides a composition for impeding the oxidation of molten nonferrous metals and alloys, such as magnesium, when such metals are exposed to oxidation conditions, such as being exposed to an oxygen-containing gas (for example air), said composition comprising *trans*-1,3,3,3-tetrafluoropropene (*trans*-HFO-1234ze).

In certain preferred embodiments, the composition further comprises at least one carrier gas selected from the group consisting of nitrogen, carbon dioxide, air, noble gas, and mixtures thereof.

The term "HFO-1234" is used herein to refer to all tetrafluoropropenes. Among the tetrafluoropropenes are included 1,1,1,2-tetrafluoropropene (HFO-1234yf) and both *cis-* and *trans*-1,1,1,3-tetrafluoropropene (HFO-1234ze). The term HFO-1234ze is used herein generically to refer to 1,1,1,3-tetrafluoropropene, independent of whether it is the *cis- or trans-* form. The terms "*cis*-HFO-1234ze" and "*trans*-HFO-1234ze" are used herein to describe the *cis-* and *trans-*forms of 1,1,1,3-tetrafluoropropene respectively. The term "HFO-1234ze" therefore includes within its scope *cis*-HFO-1234ze, *trans*-HFO-1234ze and all combinations and mixtures of these.

The term "HFCO-1233" is used herein to refer to all trifluoro-monochloropropenes. Among the trifluoro-monochloropropenes are included 1,1,1-trifluoro-2-chloro-propene (HFCO-1233xf) and both *cis-* and *trans-* 1,1,1-trifluoro-3-chloropropene (HFCO-1233zd). The term HFCO-1233zd is used herein generically to refer to 1,1,1-trifluoro-3-chloropropene, independent of whether it is the *cis-* or *trans-* form. The terms "*cis*-HFCO-1233zd" and "*trans*-HFCO-1233zd" are used herein to describe the *cis-* and *trans-* forms of 1,1,1-trifluoro-3-chloropropene, respectively. The term "HFCO-1233zd" therefore includes within its scope *cis*-HFCO-1233zd, *trans*-HFCO-1233zd, and all combinations and mixtures of these.

The term "HFO-1225" is used herein to refer to all pentafluoropropenes. Among such molecules are included 1,1,1,2,3-pentafluoropropene (HFO-1225ye), both *cis- and trans-* forms thereof. The term HFO-1225ye is thus used herein generically to refer to 1,1,1,2,3-pentafluoropropene, independent of whether it is the *cis-* or *trans-* form. The term "HFO-1225ye" therefore includes within its scope *cis*-HFO-1225ye, *trans*-HFO-1225 ye, and all combinations and mixtures of these.

The present invention provides also methods and systems which utilize the compositions of the present invention, including methods and systems for preventing oxidation of molten nonferrous metals.

As used herein, the term "air" means either ambient air, dry air, or moist air. Such compounds advantageously have an exceptionally low GWP potential, a relatively low boiling point, and are relatively non-toxic.

In addition, this invention relates to a molten metal composition comprising a nonferrous reactive metal having a protective film on its surface that is formed by a reaction between the metal and a fluoroolefin composition of the present invention, preferably said fluoroolefin composition being effective under the intended circumstances to at least partially passivate the surface of the metal, thereby reducing the chemical reactivity of the metal, especially the metal's oxidative activity.

In certain preferred embodiments, the metal is selected from the group consisting of magnesium, aluminium, lithium, and alloys of at least one of these.

According to another aspect of the present invention, provided is a method for impeding the oxidation of a molten nonferrous metal exposed to and oxygen-containing gas, such air, comprising: (a) providing molten nonferrous metal, such as magnesium, having a surface; (b) exposing said surface to a layer of gaseous fluoroolefin composition of the present invention, which is a gas containing *trans*-HFO-1234ze; and (c) forming an oxidized film on said surface. In certain preferred aspects of the method, the exposed surface of the molten reactive metal is exposed to or contacted with the gaseous fluoroolefin composition. Without being bound by or to any particular theory of operation, it is believed that the fluoroolefin composition in preferred embodiments reacts with the metal to produce an oxidatively stable film on its surface. By forming this film, the oxygen in the air can be effectively separated from the surface of the molten reactive metal and thus prevent or at least substantially inhibit the oxidation of the metal by the oxygen.

## DETAILED DESCRIPTION OF THE EMBODIMENTS OF THE INVENTION

The fluoroolefins compositions of the present invention are generally effective as cover gases to impede the oxidation of molten reactive metals when the surface of the metal is exposed to source of oxygen, such as air. As used herein, the tern "nonferrous reactive metal" means a metal or alloy which is sensitive to destructive, vigorous oxidation when exposed to air, such as magnesium, aluminum, or lithium, or an alloy comprising at least one of these metals. For convenience, the following description of illustrative embodiments of the invention shall refer to magnesium. It is under stood, however, that the present invention can also be used with aluminum, lithium, or other nonferrous reactive metal, or an alloy containing at least one of these metals.

Without necessarily being bound by theory, it is believed that by impeding oxidation, the cover gas composition of the present invention is capable of protecting the molten metal from ignition. As is the case with known fluorine-containing cover gases, it is believed that the fluoroolefin compositions of the present invention can react with the molten metal surface to create a thin passivation layer or film that can function as a barrier between the metal and an oxygen source. In contrast to conventional fluorine compounds that are used in cover gases, the fluoroolefin of the present invention is particular advantageous in that it has a relatively low GWP and a relatively low atmospheric lifetime, while also being non-toxic, effective at low concentrations, and has a low boiling point.

The compositions of the present invention comprise *trans*-HFO-1234ze.

Fluoroolefin compositions of the present invention may include a mixture of *trans*-HFO-1234ze and, optionally, a carrier gas. Preferred carrier gases include, but are not limited to, nitrogen, carbon dioxide, air, and/or noble gas such as argon. Preferably, the composition comprises a minor amount of *trans*-HFO-1234ze and a major amount of a carrier gas. In certain preferred embodiments, the composition comprises from about 0.01 to about 2 weight percent of *trans*-HFO-1234ze and from about 99.99 to about 98 weight percent of a carrier gas.

As used herein, "GWP" is a relative measure of the warming potential of a compound based on the structure of the compound. The concept of GWP was developed to compare the ability of each greenhouse gas to trap heat in the atmosphere relative to another gas. Generally, the GWP for a particular greenhouse gas is the ratio of heat trapped by one unit mass of the greenhouse gas to that of one unit mass of $CO_2$ over a specified time period. More specifically, the GWP of a compound, as defined by the Intergovernmental Panel on Climate Change (IPCC) in 1990 and updated in Scientific Assessment of Ozone Depletion: 1998 (World Meteorological Organization, Scientific Assessment of Ozone Depletion: 1998, Global Ozone Research and Monitoring Protect-Report No. 44, Geneva, 1999), is calculated as the warming due to the release of 1 kilogram of a compound relative to the warming due to the release of 1 kilogram of $CO_2$ over a specified integration time horizon (ITH):

$$GWP_X(t') = \frac{\int_0^{t'} F_X \exp(-t/\tau_X)\, dt}{\int_0^{t'} F_{CO_2} R(t)\, dt}$$

where F is the radiative forcing per unit mass of a compound (the change in the flux of radiation through the atmosphere due to the IR absorbance of that compound), C is the atmospheric concentration of a compound, $\tau$ is the atmospheric lifetime of a compound, t is time, and x is the compound of interest.

The commonly accepted ITH is 100 years representing a compromise between short-term effects (20 years) and longer-term effects (500 years or longer). The concentration of an organic compound, x, in the atmosphere is assumed to follow pseudo first order kinetics (i.e., exponential decay). The concentration of $CO_2$ over that same time interval incorporates a more complex model for the exchange and removal of $CO_2$ from the atmosphere (the Bern carbon cycle model).

The cover gas compositions of the present invention preferably include those compositions wherein the fluoroolefin compound included therein has a GWP of less than about 1000, more preferably less that about 150 and even more preferably of less than about 100. In certain preferred embodiments, each component present in the composition in a substantial amount has a GWP of less than about 1000, more preferably less that about 150 and even more preferably of less than about 100. In certain highly preferred embodiments, each component of the composition which is present in more than an insubstantial amount has a GWP of less than about 10, and even more preferably less than about 5. For comparison, the GWP of $CO_2$, certain conventional cover gases, and certain cover gases are shown in Table A.

| TABLE A | | | |
|---|---|---|---|
| Compound | GWP (100 Yr) | Atmospheric Lifetime (Yr) | Boiling Point (°C) |
| $CO_2$ | 1 | 100 - 150 | -78 |
| $SF_6$ 123,900 | | 3,200 | -82 |
| $NF_3$ | 10,800 | 740 | -121 |
| $C_2F_6$ | 11,400 | 10,000 | -78 |
| HFC-134a | 1600 | 13.6 | -26 |
| HFC-152a | 140 | 1.5 | -25 |
| HFE-7100 | 320 | 4.1 | |
| $SO_2F_2$ | 0.1 | dissipates quickly via hydrolysis and photodegradation | -55 |
| $C_2F_5C(O)CF(CF_3)_2$ | 10 | 0.1 | 49 |
| HFO-1234yf | 4 | 0.04 | -30 |
| *trans*-HFO-1234ze | 6 | 0.05 | -18.4 |
| *cis*-HFO-1234ye | <15 | <0.1 | +2 |
| HFCO-1233xf | <20 | <0.1 | +12 |
| *cis*-HFCO-1233zd | <20 | <0.1 | +19 |
| *trans*-HFCO-1233zd | <20 | <0.1 | +19 |

Preferably, the cover gas compositions of the present invention include those compositions wherein the fluoroolefin component has a atmospheric lifetime of less than about 20 (years), preferably less than about 10 (years), and even more preferably less than about 1 (year). As used herein the term "atmospheric lifetime" is the approximate amount of time it would take for the concentration of the compound to fall to $e^{-1}$ of its initial value as a result of either being converted into another chemical compound (wherein e is the base of natural logarithms). Atmospheric lifetime is closely related to GWP since relatively short lifetimes limit the duration that a reactant can participate in a reaction.

In addition, preferred cover gas compositions of the present invention comprise what are more compounds wherein each compound present in more than an insubstantial amount has a boiling point of less than about 25°C, and even more preferably less than about 0° C. Cover gases that have boiling points close to or above room temperature (i.e. which are liquids at room temperature) typically require additional metering equipment to disperse the cover gas material in a controlled fashion onto the surface of the molten metal.

Preferably, the fluoroolefin used in the present compositions has low or no toxicity. In this regard, it is preferred that the fluoroolefin component that is present in the compositions in more than an insubstantial amount has a LC-50 value of at least about 100,000 ppm, and more preferably at least about 200,000 ppm. As used herein, the term "LC-50 value" means the concentration of the fluoroolefin in air that will kill 50% of test subject (e.g. mice) when administered as a single exposure (e.g. 4 hours). HFC-1234ze has been found to have a 4-hour LC-50 of at least 100,000, and HFC-1234yfhas been found to have a 4-hour LC-50 of at least about 200,000. For comparison, $C_2F_5C(O)CF(CF_3)_2$ (a fluor-

oketone cover gas marketed by Minnesota Mining and Manufacturing Co. of St. Paul, Minnesota, under the tradename Novec™) has a 4-hour LC-50 of about 100,000. Other compounds, such as sulfuryl fluoride, nitrosyl fluoride, and nitrogen trifluoride are know to be toxic and/or hazardous materials.

Another measure of toxicity is a compound's No Observed Adverse Effect Level (NOAEL). As used herein, the term NOAEL refers to the greatest concentration or amount of a substance, found by experiment or observation, which causes no detectable adverse alteration of morphology, functional capacity, growth, development, or life span of the target organism under defined conditions of exposure. For cardiac sensitization tests, the NOAEL for HFO-1234yf and HFO-1234ze are greater than 12 vol. %. By comparison, the NOAEL for $C_2F_5C(O)CF(CF_3)_2$ is only 10 vol. %.

Applicants have found that different isomeric forms of certain fluoroolefins do not possess the same advantageous characteristics for cover gas applications. For example, among isomers of HFO-1225, the HFO-1225zc isomer is much more toxic, and thus less preferred, than the HFO-1225ye or HFO-1225yc isomer. In certain preferred embodiments, the cover gas consists essentially of only the *trans*-isomer of HFO-1234ze.. The *trans*-isomer of HFO-1234ze can be utilized in the present invention with much greater success than the related *cis*-isomer or than mixtures of the *cis*- and *trans*- isomers. In particular, the *trans*-isomer is more preferred not only because is less toxic than the *cis*-isomer, but also because it has a lower normal boiling point (-18.4° C vs. 9° C for *trans*- and *cis*-isomers, respectively). This low boiling point correlates to a higher vapor pressure of the gas which is advantageous in that the gas is more easily metered as it is applied to a molten metal. Isomeric mixtures of the *cis*-and *trans*- isomers can be problematic because the isomers do not have the same vapor pressure, and thus are not evenly dispensed from a container. That is, dispersement of the isomeric mixture from a container will initially result in a cover gas having a higher concentration of the lower boiling isomer and will eventually result in a cover gas having a higher concentration of the higher boiling isomer. Such a mixture makes it more difficult to maintain a steady flow and composition.

## EXAMPLES

Certain aspects of the present invention are further illustrated, but is not limited by, the following examples.

Examples 1 - 5 demonstrate the efficacy of the fluoroolefin as a Mg covergas according to the present invention.

### Example 1:

A quartz tube having a well was equipped with a metered source of cover gas and a thermocouple which was placed in the well. The well was filled with about 0.2 to 0.3 g of solid magnesium pieces. The cover gas was a mixture of air (a carrier gas) and *trans*-HFO-1234ze. The air and the *trans*-HFO-1234ze were provided from separate cylinders and the relative amounts of each entering the mixture were controlled to give composition of about 4.5% *trans*-HFO-1234ze by volume.

The tube containing the magnesium was placed in an oven. A flow of cover gas through the tube and over the well containing the magnesium was then established at about 1 liter/ minute. The oven was then heated to about 700°C. The flow of cover gas proceeded until a surface film was formed on the magnesium or the magnesium ignited.

After the test was complete, the magnesium was removed from the oven and visually inspected to determine the quality of the cover gas.

The magnesium contained a white coating (presumably MgO or $MgF_2$) indicating that the magnesium was well protected.

### Example 2:

The experiment of Example 1 was repeated, except that the cover gas contained about 1.5% *trans*-HFO-1234ze by volume.

The magnesium contained a white coating and the pieces were not stuck together indicating that the magnesium was well protected.

### Example 3:

The experiment of Example 1 was repeated, except that the cover gas contained about 0.5% trans-HFO-1234ze by volume.

The magnesium contained a white coating and the pieces were not stuck together indicating that the magnesium was well protected.

**Example 4:**

The experiment of Example 1 was repeated, except that the cover gas contained about 0.2% *trans-HFO*-1234ze by volume.

The magnesium contained a white coating with some dark spots and the pieces were not stuck together indicating that the magnesium was well protected.

**Example 5:**

The experiment of Example 1 was repeated, except that the cover gas contained about 0.1% *trans*-HFO-1234ze by volume.

The magnesium contained a white coating with a few brown specks indicating that the magnesium was protected in general.

**Comparative Examples:**

The experiments of Examples 1 - 5 were repeated, except that the cover gas contained about either $SF_6$ or HFC-134a.

The results of the comparative examples are provided in Table B. In general, *trans*-HFO-1234ze, $SF_6$, and HFC-134a performed well as cover gases at concentrations at or above about 1.5% by volume. However, performance of the different cover gases began to vary at about 0.5% by volume, with HFC-134a performing better than $SF_6$, and *trans*-HFO-1234ze performing better than HFC-134a. It is believed that the ability of the cover gas to protect the magnesium, and particularly to keep the magnesium from igniting, corresponds to the amount of fluorine it provides to create a protective barrier. Thus, cover gases that are more reactive, such as *trans*-HFO-1234ze, are better suited to protect magnesium compared to more stable gases, such as $SF_6$.

TABLE B

| Vol. % of F-Source in Air | F-Source | Quality of Mg Protection |
|---|---|---|
| 4.5 | SFe | white coating; pieces not stuck together |
| 4.5 | HFC-134a | white coating; pieces not stuck together |
| 4.5 | *trans*-HFO- 1234z | white coating; pieces not stuck together |
| 1.5 SF$_6$ | | white coating; pieces not stuck together |
| 1.5 | HFC-134a | white coating; pieces not stuck together |
| 1.5 | *trans*-HFO- 1234ze | white coating; pieces not stuck together |
| 0.43 | SFe | coating less white; brownish regions; Mg maintained partial luster |
| 0.60 | HFC-134a | white coating with no brown spots |
| 0.46 | *trans*-HFO- 1234ze | white coating with no brown spots |
| 0.20 | SFe | several brownish regions, very little luster, Mg pieces stuck together |
| 0.18 | HFC-134a white with brown spots, a couple of pieces stuck together | |
| 0.26 | *trans*-HFO-1234z white with dark spots, no pieces stuck together | |
| 0.11 | SFe | failure; Mg ignited |
| 0.10 | HFC-134a | most brown specks, protected in general |
| 0.09 *trans*-HFO- 1234ze | | a few brown specks, well protected in general |

Having thus described a few particular embodiments of the invention, various alterations, modifications, and improvements will readily occur to those skilled in the art. Such alterations, modifications, and improvements, as are made obvious by this disclosure, are intended to be part of this description though not expressly stated herein, and are intended to be within the spirit and scope of the invention. Accordingly, the foregoing description is by way of example only, and not limiting. The invention is limited only as defined in the following claims and equivalents thereto.

**Claims**

1. A cover gas composition for impeding the oxidation of molten nonferrous metals and alloys when exposed to air, said composition comprising trans-1,3,3,3-tetrafluoropropene (trans-HFO-1234ze).

2. The cover gas composition of claim 1 wherein said composition further comprises at least one carrier gas selected from the group consisting of nitrogen, carbon dioxide, air, noble gas, and mixtures thereof.

3. A method for impeding the oxidation of a molten nonferrous metal exposed to air, comprising:

   (a) providing molten nonferrous metal having a surface;
   (b) exposing said surface to a layer of gaseous fluoroolefin composition;
   (c) forming an oxidized film on said surface,

   wherein said fluoroolefin composition contains trans-1,3,3,3-tetrafluoropropene (trans-HFO-1234ze).

4. A molten metal composition comprising a nonferrous reactive metal having a protective film on its surface, wherein said film is formed by a reaction between the metal and a fluoroolefin composition as defined in any of claims 1 to 2, and said film impedes the oxidation of said metal.

5. The molten metal composition of claim 4, wherein said metal is selected from the group consisting of magnesium, aluminum, lithium, and alloys of at least one these.

**Patentansprüche**

1. Schutzgaszusammensetzung zur Verhinderung der Oxidation von geschmolzenen Nichteisenmetallen und Legierungen, wenn diese der Luft ausgesetzt sind, wobei die Zusammensetzung trans-1,3,3,3-Tetrafluorpropen (trans-HFO-1234ze) umfasst.

2. Schutzgaszusammensetzung nach Anspruch 1, wobei die Zusammensetzung weiter mindestens ein Trägergas umfasst, ausgewählt aus der Gruppe bestehend aus Stickstoff, Kohlendioxid, Luft, Edelgas und Mischungen davon.

3. Verfahren zur Verhinderung der Oxidation eines geschmolzenen Nichteisenmetalls, das der Luft ausgesetzt ist, umfassend:

   (a) Bereitstellen von geschmolzenem Nichteisenmetall, das eine Oberfläche aufweist;
   (b) Aussetzen der Oberfläche einer Schicht einer gasförmigen Fluoroolefinzusammensetzung;
   (c) Bilden eines oxidierten Films auf der Oberfläche,

   wobei die Fluoroolefinzusammensetzung trans-1,3,3,3-Tetrafluorpropen (trans-HFO-1234ze) enthält.

4. Geschmolzene Metallzusammensetzung, umfassend ein reaktives Nichteisenmetall, das einen Schutzfilm auf seiner Oberfläche aufweist, wobei der Film durch eine Reaktion zwischen dem Metall und einer Fluoroolefinzusammensetzung gemäß einem der Ansprüche 1 bis 2 gebildet wird und der Film die Oxidation des Metalls verhindert.

5. Geschmolzene Metallzusammensetzung nach Anspruch 4, wobei das Metall ausgewählt ist aus der Gruppe bestehend aus Magnesium, Aluminium, Lithium und Legierungen von mindestens einem derselben.

**Revendications**

1. Composition de gaz de couverture permettant de prévenir l'oxydation de métaux non ferreux en fusion et d'alliages lorsqu'ils sont exposés à l'air, ladite composition comprenant du trans-1,3,3,3-tétrafluoropropène (trans-HFO-1234ze).

2. Composition de gaz de couverture selon la revendication 1, dans laquelle ladite composition comprend en outre au moins un gaz vecteur choisi dans le groupe constitué de l'azote, du dioxyde de carbone, de l'air, d'un gaz rare et de mélanges de ceux-ci.

3. Procédé de prévention de l'oxydation d'un métal non ferreux en fusion exposé à l'air, comprenant :

   (a) la fourniture d'un métal non ferreux en fusion présentant une surface ;
   (b) l'exposition de ladite surface à une couche de composition gazeuse de fluorooléfine ;
   (c) la formation d'un film oxydé sur ladite surface,

   dans lequel ladite composition de fluorooléfine contient du trans-1,3,3,3-tétrafluoropropène (trans-HFO-1234ze).

4. Composition de métal en fusion comprenant un métal réactif non ferreux présentant un film protecteur sur sa surface, dans laquelle ledit film est formé par une réaction entre le métal et une composition de fluorooléfine telle que définie dans l'une quelconque des revendications 1 à 2 et ledit film préient l'oxydation dudit métal.

5. Composition de métal en fusion selon la revendication 4, dans laquelle ledit métal est choisi dans le groupe constitué du magnésium, de l'aluminium, du lithium et des alliages d'au moins un de ceux-ci.

EP 2 038 439 B3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 1972317 A, Reimers
- WO 0064614 A
- US 6521018 B, Hobbs
- US 6537346 B
- US 6685764 B
- US 6780220 B, Milbrath
- US 6929674 B
- EP 0964845 A
- US 5710352 A
- WO 2004037752 A

### Non-patent literature cited in the description

- **D. MILBRATH.** Development of 3M Novec 612 Magnesium Protection Fluid as a Substitute for SF6 over Molten Magnesium. *International Conference on SF6 and the Environment,* 21 November 2002, www.epa.gov/highgwp/elec tricpowerw-sf6/pdf/mil-brath.pdf.
- *Scientific Assessment of Ozone Depletion,* 1998
- Scientific Assessment of Ozone Depletion. Global Ozone Research and Monitoring Protect-Report No. 44. World Meteorological Organization, 1998